# EUROPEAN PATENT SPECIFICATION

(11) **EP 2 891 500 B1**
(45) Date of publication and mention of the grant of the patent: **01.08.2018**
(21) Application number: 14150267.4
(22) Date of filing: 07.01.2014
(51) Int. Cl.: A61K 49/10

(54) **Contrast agent for determination of aldehyde dehydrogenase (ALDH) activity**
Kontrastmittel zur Bestimmung von Aldehyd-Dehydrogenase-(ALDH)-Aktivität
Agent de contraste pour la détermination de l'activité d'aldéhyde déshydrogénase (ALDH)

(43) Date of publication of application: 08.07.2015
(73) Proprietor: Cambridge Enterprise Limited, Cambridge, Cambridgeshire CB2 1TN (GB)
(72) Inventor: Brindle, Kevin, Cambridge, Cambridgeshire CB2 0RE (GB); Kettuen, Mikko, Cambridge, Cambridgeshire CB2 0RE (GB); Dzien, Piotr, Cambridge, Cambridgeshire CB2 0RE (GB)
(74) Representative: Zacco Norway AS

(56) References cited:
- THOMAS TRANTZSCHEL ET AL: "Parahydrogen induced polarization in face of keto-enol tautomerism: proof of concept with hyperpolarized ethanol", PHYSICAL CHEMISTRY CHEMICAL PHYSICS, vol. 14, no. 16, 1 January 2012 (2012-01-01), page 5601, XP055112792, ISSN: 1463-9076, DOI: 10.1039/c2cp40272f
- DANIEL M. SPIELMAN ET AL: "In vivo measurement of ethanol metabolism in the rat liver using magnetic resonance spectroscopy of hyperpolarized [1- 13 C]pyruvate", MAGNETIC RESONANCE IN MEDICINE, vol. 62, no. 2, 1 August 2009 (2009-08-01) , pages 307-313, XP55112860, ISSN: 0740-3194, DOI: 10.1002/mrm.21998
- YUN XIANG ET AL: "In vivo detection of intermediate metabolic products of [1-13C]ethanol in the brain using 13C MRS", NMR IN BIOMEDICINE, vol. 24, no. 9, 11 February 2011 (2011-02-11), pages 1054-1062, XP055112824, ISSN: 0952-3480, DOI: 10.1002/nbm.1653
- J. WANG ET AL: "Oxidation of ethanol in the rat brain and effects associated with chronic ethanol exposure", PROCEEDINGS OF THE NATIONAL ACADEMY OF SCIENCES, vol. 110, no. 35, 12 August 2013 (2013-08-12), pages 14444-14449, XP55112760, ISSN: 0027-8424, DOI: 10.1073/pnas.1306011110
- Sonal Josan ET AL: "In vivo measurement of aldehyde dehydrogenase-2 activity in rat liver ethanol model using dynamic MRSI of hyperpolarized [1- 13 C]pyruvate : IN VIVO ALDH2 ACTIVITY MEASUREMENT USING HYPERPOLARIZED 13 C MRSI", NMR IN BIOMEDICINE., 1 December 2012 (2012-12-01), pages n/a-n/a, XP055370132, GB ISSN: 0952-3480, DOI: 10.1002/nbm.2897
- PIOTR DZIEN ET AL: "13 C magnetic resonance spectroscopic imaging of hyperpolarized [1- 13 C, U- 2 H 5 ] ethanol oxidation can be used to assess aldehyde dehydrogenase activity in vivo : Imaging Ethanol Oxidation", MAGNETIC RESONANCE IN MEDICINE., vol. 73, no. 5, 6 May 2015 (2015-05-06), pages 1733-1740, XP055370164, US ISSN: 0740-3194, DOI: 10.1002/mrm.25286

## Description

The present invention relates to a new composition which may be used as a contrast agent and to a method of ¹³C magnetic resonance (MR) detection which can be used to determine aldehyde dehydrogenase (ALDH) or also alcohol dehydrogenase (ADH) activity.

The metabolism of alcohol proceeds via two steps, as indicated in the schematic representation below:

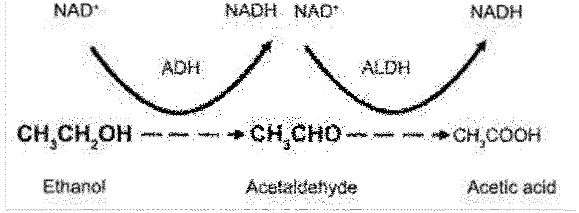

The major pathway of oxidative metabolism of ethanol involves alcohol dehydrogenase (ADH) which produces acetaldehyde. At high ethanol concentration the enzymes catalase and P4502E1 may also display some activity. Acetaldehyde is in turn metabolized to acetate by the action of aldehyde dehydrogenase (ALDH), mainly by ALDH2.

Aldehyde Dehydrogenase (ALDH2) is an emerging drug target for the treatment of heart disease, cocaine and alcohol dependence and conditions caused by genetic polymorphisms in ALDH2. Non-invasive measurement of ALDH2 activity *in vivo* could inform the development of these drugs and accelerate their translation to the clinic.

The object of the present invention is to provide a composition and a method for the determination of ALDH or ADH activity *in vitro* or *in vivo* in a human or non-human animal body.

Ethanol is oxidized by the liver to produce acetate, the majority of which exits via the hepatic vein and is metabolized further in other tissues (Lundquist, F. "Production and utilization of free acetate in man", Nature 1962; 193; 579-580). At low to medium blood concentrations of ethanol (up to 24 mM in humans) the bulk of its oxidation to acetaldehyde and further to acetate is via the coupled reactions catalyzed by the NAD⁺-dependent dehydrogenases: cytosolic alcohol dehydrogenase (ADH) and mitochondrial aldehdyde dehydrogenase (ALDH2) (Lieber, C.S. "Ethanol metabolism, cirrhosis and alcoholism",Clin.Chim. Acta 1997; 257: 59-84; Korsten, M.A., Matsuzaki, S., Feinman, L., Lieber C.S. "High blood acetaldehyde levels after ethanol administration. Difference between alcoholic and nonalcoholic subjects", N. Engl. J. Med. 1975; 292:386-389). Microsomal NADPH-dependent cytochrome CYP2E1 and peroxisomal H₂O₂-dependent catalase can also oxidize ethanol to acetaldehyde, their relative contributions increasing, for example, during exposure to high-doses of ethanol and in starvation respectively (Lieber, C.S. "Microsomal ethanol-oxidizing systems (MEOS): the first 30 years (1968-1998) - review." Alcohol Clin. Exp. Res. 1999; 23:991-1007; Handler, J.A.; Thurman, R.G." Catalase-dependent ethanol oxidation in perfused rat liver. Requirement for fatty-acid stimulated H2O2 production by peroxisomes.", Eur.J.Biochem. 1988; 176: 477-484). Ethanol oxidation by hepatic dehydrogenases causes an acute increase in the NADH/NAD⁺ ratio, leading to an increase in cytosolic lactate concentrations and disturbances of lipid metabolism, with consequent onset of alcoholic fatty liver disease (Lieber, C.S. "Ethanol metabolism, cirrhosis and alcoholism",Clin.Chim. Acta 1997; 257: 59-84; Krebs, H.A., Freedland, R.A.; Hems, R., Stubbs, M. "Inhibition of hepatic gluconeogensis by ethanol.", Biochem.J. 1969; 112: 117-124). Adaptive induction of hepatic CYP2E1 activity by chronic ethanol exposure, which increases the liver's capacity to produce acetaldehyde from ethanol, is accompanied by a decreasing capacity of the liver mitochondria to oxidize this acetaldehyde. This decreased capacity results from functional and structural impairment caused by exposure to the elevated acetaldehyde concentrations (Lieber, C.S., DeCarli, L.M. "Ethanol oxidation by hepatic microsomes: adaptive increase after ethanol feeding." Science 1968; 162:917-918; Hasumura; Y., Teschke, R., Lieber, C.S. "Acetaldehyde oxidation by hepatic mitochondria: decrease after chronic ethanol consumption." Science 1975; 189: 727-729; Hoek, J.B., Cahill, A., Pastorino, J.G. "Alcohol and mitochondria: a dysfunctional relationship." Gastroenterology 2002; 122: 2049-2063). Inefficient disposal of acetaldehyde and its accumulation, both intra- and extra-hepatically, is the direct cause of liver cirrhosis, and is implicated in alcohol-induced cardiomyopathy and pancreatitis, and in some types of cancer, particularly in the upper aerodigestive tract (UADT) (Yokoyama, A., Omori, T. Yokoyama, T. "Alcohol and aldehyde dehydrogenase polymorphisms and a new strategy for prevention and screening for cancer in the upper aerodigestive tract in East Asians." Keio J. Med.; 59:115-130, Poschl, G., Seitz, H.K., "Alcohol and cancer." Alcohol 2004; 39: 155-165).

The capacity to oxidize endogenous and exogenous aldehydes, conferred upon extra-hepatic tissues by expression of ALDH2, is fundamental for their normal function. Pharmacological activation of ALDH2 protects the myocardium from ischemic injury by stimulating the oxidative activity of ALDH2 towards 4-hydroxy-2-nonenal, a toxic aldehyde produced in ischemic heart (Chen, C.H., Budas, G.R., Churchill, E.N., Disatnik, M.H., Hurley, T.D., Mochly-Rosen, D. "Activation of aldehyde dehydrogenase-2 reduces ischemic damage to the heart." Science 2008; 321: 1493-1495; Sun, L., Ferreira, J.C., Mochly-Rosen, D. "ALDH2 activator inhibits increased myocardial infarction injury by nitroglycerin tolerance." Sci.Transl.Med. 2011 (107): 107-111, Chen, C.H., Gray, M.O., Mochly-Rosen, D. "Cardioprotection from ischemia by a brief exposure to physiological levels of ethanol: role of epsilon protein kinase." C.Proc. Natl. Acad. Sci. USA 1999; 96: 12784-12789). ALDH2 is expressed in brain tissue, where it is essential for the maintenance of dopamine (DA) homeostasis and the oxidation of the endogenous aldehydes DOPAL (3,4-dihydroxyphenylacetaldehyde) and DOPEGAL (3,4-dihydroxyphenylglycolaldehyde). DOPEGAL, DOPAL and DA accumulation is neurotoxic and disturbances of their metabolism are implicated in neurodegenerative disorders such as Parkinson's disease (PD) and in Alzheimer's disease (AD) (Marchitti, S.A., Deitrich, R.A., Vasiliou, V. "Neurotoxicity and metabolism of the catecholamine-derived 3,4-dihydroxyphenylacetaldehyde and 3,4-dihydroxyphenylglycolaldehyde: the role of aldehyde dehydrogenase.",Pharmacol. Rev. 2007; 59: 125-150; LaVoie, M.J., Ostaszewski, B.L., Weihofen, A., Schlossmacher, M.G., Selkoe, D.J., "Dopamine covalently modifies and functionally inactivates parkin.", Nat.Med. 2005; 11: 1214-1221). In addition, DA signaling in brain plays a major role in establishing addictive behaviors and in triggering relapse (Yao, L., Fan, P., Arolfo, M., Jiang, Z., Olive, M.F., Zablocki, J., Sun, H.L., Chu, N., Lee, J., Kim, H.Y., Leung, K., Shryock, J., Blackburn, B., Diamond, I., "Inhibition of aldehyde dehydrogenase-2 suppresses cocaine seeking by generating THP, a cocain use-dependent inhibitor of dopamine synthesis.", Nat. Med. 2010; 16: 1024-1028; Arolfo, M.P., Overstreet, D.H., Yao, L., Fan, P., Lawrence, A.J., Tao, G., Keung, W.M., Vallee, B.L., Olive, M.F., Gass, J.T., Rubin, E., Anni, H., Hodge, C.W., Besheer, J., Zablocki, J., Leung, K., Blackburn, B.K., Lange, L.G., Diamond, I., "Suppression of heavy drinking and alcohol seeking by a selective ALDH-2 inhibitor.", Alcohol Clin.Exp.Res., 2009; 33: 1935-1944).

The role of ALDH2 in human health is illustrated by the inability of individuals carrying the ALDH2*2 allele, which encodes an inactive E487K mutant, to dispose efficiently of toxic aldehydes. ALDH2*2 allele carriers are at higher risk of increased morbidity after myocardial infarction and are more likely to develop late-onset AD and cancers in the UADT (Yokohama, A., Omori, T., Yokohama T., "Alcohol and aldehyde dehydrogenase polymorphisms and a new strategy for prevention and screening for cancer in the upper aerodigestive tract in East Asians.", Keio J. Med.; 59:115-130; Chen, C.H., Budas, G.R., Churchill, E.N., Disatnik, M.H., Hurley, T.D., Mochly-Rosen, D., "Activation of aldehyde dehydrogenase-2 reduces ischemic damage to the heart.", Science 2008; 321: 1493-1495; Kamino, K., Nagasaka, K., Imagawa, M., Yamamoto, H., Yoneda, H., Ueki, A., Kitamura, S., Namekata, K., Miki, T., Ohta, S., "Deficiency in mitochondrial aldehyde dehydrogenase increases the risk for late-onset Alzheimer's disease in the Japanese population.", Biochem.Biophys.Res.Commun. 2000; 273: 192-196; Perez-Miller, S., Younus, H., Vanam, R., Chen, C.H., Mochly-Rosen, D., Hurley, T.D., " Alda-1 is an agonist and chemical chaperone for the common human aldehyde dehydrogenase 2 variant.", Nat. Struct. Mol. Bio 2010; 17:159-164). The number of ALDH2*2 allele carriers worldwide is estimated to be at least 540 million, comprising approximately 40% of native East Asian populations (Perez-Miller, S., Younus, H., Vanam, R., Chen, C.H., Mochly-Rosen, D., Hurley, T.D., " Alda-1 is an agonist and chemical chaperone for the common human aldehyde dehydrogenase 2 variant.", Nat. Struct. Mol. Bio 2010; 17:159-164).

The recently developed ALDH2 activator, Alda-1, was shown to confer protection against myocardial ischemic damage in a rat model of infarction (Chen, C.H., Budas, G.R., Churchill, E.N., Disatnik, M.H., Hurley, T.D., Mochly-Rosen, D., "Activation of aldehyde dehydrogenase-2 reduces ischemic damage to the heart.", Science 2008; 321: 1493-1495; Sun, L., Ferreira, J.C., Mochly-Rosen, D., "ALDH2 activator inhibits increased myocardial infarction injury by nitroglycerin tolerance.", Sci.Transl.Med. 2011 (107): 107-111) and a ALDH2- specific inhibitor, CVT-10216, inhibited addictive substance seeking and relapse in rat models of cocaine addiction and alcoholism (Yao, L., Fan, P., Arolfo, M., Jiang, Z., Olive, M.F., Zablocki, J., Sun, H.L., Chu, N., Lee, J., Kim, H.Y., Leung, K., Shryock, J., Blackburn, B., Diamond, I., "Inhibition of aldehyde dehydrogenase-2 suppresses cocaine seeking by generating THP, a cocain use-dependent inhibitor of dopamine synthesis.", Nat. Med. 2010; 16: 1024-1028; Arolfo, M.P., Overstreet, D.H., Yao, L., Fan, P., Lawrence, A.J., Tao, G., Keung, W.M., Vallee, B.L., Olive, M.F., Gass, J.T., Rubin, E., Anni, H., Hodge, C.W., Besheer, J., Zablocki, J., Leung, K., Blackburn, B.K., Lange, L.G., Diamond, I., "Suppression of heavy drinking and alcohol seeking by a selective ALDH-2 inhibitor.", Alcohol Clin.Exp.Res. 2009; 33: 1935-1944).

In addition, the catalytic activity of the E487K ALDH2 mutant can be partially restored by Alda-1, which could have important implications for clinical management of diseases affecting carriers of the ALDH2*2 allele (Perez-Miller, S., Younus, H., Vanam, R., Chen, C.H., Mochly-Rosen, D., Hurley, T.D., "Alda-1 is an agonist and chemical chaperone for the common human aldehyde dehydrogenase 2 variant.", Nat. Struct. Mol. Biol. 2010; 17: 159-164). A non-invasive method for measuring ALDH activity *in vivo* would inform preclinical development of these drugs and could accelerate their translation to the clinic.

Much of what is known about ethanol metabolism derives from *ex vivo* studies on perfused organs, isolated hepatocytes and tissue samples (Krebs, H.A., Freedland, R.A., Hems, R., Stubbs, M., "Inhibition of hepatic gluconeogenesis by ethanol.", Biochem.J. 1969; 112: 117-124; Yao, C.T., Lai, C.L., Hsieh, H.S., Chi, C.W., Yin, S.J., "Establishment of steady-state metabolism of ethanol in perfused rat liver: the quantitative analyses using kinetic mechanism-based rate equations of alcohol dehydrogenase.", Alcohol 2010; 44: 541-551; Klyosov, A.A., Rashkovetsky, L.G., Tahir, M.K., Keung, W.M., "Possible role of liver cytosolic and mitochondrial aldehyde dehydrogenases in acetaldehyde metabolism.", Biochemistry 1996; 35: 4445-4456). Investigation of ethanol metabolism *in vivo* has been limited to magnetic resonance spectroscopy (MRS) studies, which have been used, for example, to investigate the distribution of ¹³C label in rat brain metabolite pools following systemic administration of [1-¹³C] ethanol (Xiang, Y., Shen, J., "In vivo detection of intermediate products of [1-13C]ethanol in the brain using 13C MRS.", NMR Biomed. 2011; 24: 1054-1062). The main limitation of using ¹³C MRS in this way is its low sensitivity, which results in relatively low temporal and spatial resolution.

The recent introduction of dissolution dynamic nuclear polarization (DNP) (Ardenkjaer-Larsen, J.H., Fridlund, B., Gram, A., Hansson, G., Hansson, L., Lerche, M.H., Servin, R., Thaning, M., Golman, K., "Increase in signal-to-noise ratio of >10,000 times in liquid-state NMR.", Proc. Natl. Acad. Sci. USA 2003; 100: 10158-10163), which can increase the sensitivity of ¹³C MRS by at least four orders of magnitude, has made possible measurements of the metabolism of many ¹³C-labeled substrates *in vivo* (Gallagher, F.A., Kettunen, M.I., Brindle; K.M., "Biomedical applications of hyperpolarised 13C magnetic resonance imaging.", Progress in Nuc. Mag. Res. Spec. 2009; 55: 285-295; Kurhanewicz, J., Vigneron, D.B., Brindle, K.M., Chekmenev; E.Y, Comment, A., Cunningham, C.H., Deberardinis, R.J., Green, G.G., Leach, M.O., Rajan, S.S., Rizi, R.R., Ross, B.D., Warren, W.S., Malloy, C.R., "Analysis of cancer metabolism by imaging hyperpolarised nuclei: prospects for translation to clinincal research.", Neoplasia 2011; 13: 81-97). The activity of ALDH2 has been estimated indirectly from measurements of hyperpolarised ¹³C label exchange between injected [1-¹³C]pyruvate and endogenous lactate, in the reaction catalyzed by lactate dehydrogenase (LDH) (Josan, S., Spielman, D., Yen, Y.F., Hurd, R., Pfefferbaum, A., Mayer, D., "Fast volumetric imaging of ethanol metabolism in rat liver with hyperpolarised [1-13C]pyruvate.", NMR Biomed. 2012; 25: 993-999; Spielman, D.M., Mayer, D., Yen, Y.F., Tropp, J., Hurd, R.E., Pfefferbaum, A., "In vivo measurement of ethanol metabolism in the rat liver using magnetic resonance spectroscopy of hyperpolarised [1-13C]pyruvate.", Magn.Reson.Med. 2009; 62: 307-313). This exchange was increased in rat liver by ethanol injection, which increased the NADH/NAD+ ratio, and was decreased by the ALDH2 inhibitor, disulfiram. An increase in the NADH/NAD+ ratio strongly stimulates the LDH-catalyzed exchange (Witney, T.H., Kettunen, M.I., Brindle, K.M., "Kinetic modeling of hyperpolarised 13C label between pyruate and lactate in tumor cells.", J.Biol.Chem. 2011; 286: 24572-24580). However, this indirect measurement assumes that during ethanol metabolism the change in the mitochondrial NADH/NAD⁺ ratio mirrors its cytosolic equivalent (Josan, S., Xu, T., Yen, Y.F., Hurd, R., Ferreira, J., Chen, C.H., Mochly-Rosen, D., Pfefferbaum, A., Mayer, D., Spielman, D., "In vivo measurement of aldehyde dehydrogenase-2 activity in rat liver ethanol model using dynamic MRSI of hyperpolarised [1-13C]pyruvate.", NMR Biomed. 2013; 26: 607-612). This is not the case, for example, in physiological states where the relative contribution of NAD⁺-independent pathways of ethanol oxidation to acetaldehyde, such as that catalyzed by CYP2E1, become important (Korsten, M.A., Matsuzaki, S., Feinman, L., Lieber, C.S., "High blood acetaldehyde levels after ethanol administration. Difference between alcoholic and non-alcoholic subjects.", N. Engl. J.Med. 1975; 292: 386-389).

Thomas Trantzschel et. al discloses in Phys. Chem.Chem.Phys., 2012, vol. 14, pages 5601-5604 parahydrogen induced polarization in face of keto-enol tatorism: proof of concept with hypepolarized ethanol.

Sonal Josan et al. discloses in NMR in Biomedicine, 1. December 2012, XP055370132 *in vivo* measurement of aldehyde dehydrogenase-2 activity in rat liver ethanol model using dynamic MRSI of hyperpolarized [1-13C]pyruvate.

The present invention adresses the need for new improved compositions and methods for determining ALDH activity or ADH activity, especially ALDH-2 activity *in vitro* or *in vivo.*

It has thus been demonstrated that oxidation of hyperpolarised perdeuterated [1-¹³C] ethanol ([1-¹³C, U-²H₅]ethanol) to [1-¹³C] acetate, in the coupled reactions catalyzed by cytosolic ADH and mitochondrial ALDH2, can be measured and imaged directly in mouse liver *in vivo* using ¹³C MRS/magnetic resonance spectroscopic imaging (MRSI).

The influence of ALDH2 activity on this label flux was demonstrated by inhibiting the enzyme with the irreversible inhibitor, disulfiram (Klyosov, A.A., Rashkovetsky, L.G., Tahir, M.K., Keung, W.M., "Possible role of liver cytosolic and mitochondrial aldehyde dehydrogenase in acetaldehyde metabolism.", Biochemistry 1996; 35: 4445-4456).

The present invention thus provides a composition comprising hyperpolarised [¹³C]ethanol, which ethanol is deuterated on at least one hydrogen atom, wherein the hyperpolarisation is achieved on the NMR active ¹³C- nuclei.

The said composition is preferably a MR contrast agent.

In a preferred embodiment of the invention carbon 1 of the ethanol is ¹³C-labelled.

In a further embodiment of the invention carbon 2 of the ethanol is ¹³C-labelled.

In a still further embodiment of the invention both carbon 1 and carbon 2 of the ethanol are ¹³C-labelled.

A further embodiment of the invention is a composition comprising hyperpolarised [¹³C]ethanol, wherein said ethanol is deuterated on at least two hydrogen atoms.

In a still further embodiment of the invention, the ethanol is deuterated on at least three hydrogen atoms.

In a further embodiment of the invention, the ethanol is deuterated on at least four hydrogen atoms.

In still another embodiment of the invention, the ethanol is deuterated on five hydrogen atoms.

In a further preferred embodiment the hydroxy hydrogen is not deuterated.

A preferred embodiment of the invention comprises a composition wherein the deuterated [¹³C]ethanol is [1-¹³C, U-²H₅]. U indicates that the hydrogen is uniformly labeled.

The invention further comprises a composition as described above for use in a method for determining ALDH activity, wherein the signal of ¹³C-acetate and optionally the signal of ¹³C-ethanol and/or ¹³C-acetaldehyde is/are detected by ¹³C-MR detection.

In another embodiment the signal of ¹³C-acetate is determined.

In another embodiment the signal of ¹³C-acetaldehyde is determined.

In still another embodiment the signal of ¹³C-ethanol is determined.

Aldehyde dehydrogenase ALDH comprises a number of families, including ALDH1, ALDH2 and ALDH3.

According to a preferred embodiment the use is in a method for determining ALDH2 activity.

According to a further embodiment the use is in a method for determining ALDH1 activity.

According to a still further embodiment the use is in a method for determining ALDH3 activity.

Another embodiment of the invention is a composition for use in an *in vivo* method for determining ALDH activity.

Still another embodiment of the invention is a composition for use in an *in vivo* method for determining ADH activity.

The method is preferably a method for determining ALDH or ADH activity in a human or non-human animal body.

Still another embodiment of the invention is a composition for use in an *in vitro* method for determining ALDH activity.

Another embodiment of the invention is a composition for use in an *in vitro* method for determining ADH activity.

The method is preferably a method for determining ALDH or for determining ADH activity in a cell culture, in body samples or in isolated organs derived from a human or non-human animal body.

Another embodiment of the present invention is a composition as described above, for use in a method for determining ALDH activity, wherein the signal of ¹³C-acetate and the signal of ¹³C-ethanol are detected by ¹³C-MR detection.

Still another embodiment of the present invention is a composition as described above for use in a method for determining ALDH activity, wherein the signal of ¹³C-acetate and the signal of ¹³C-acetaldehyde are detected by ¹³C-MR detection.

Still another embodiment of the present invention is a composition as described above for use in a method for determining ALDH activity, wherein the signal of ¹³C-acetate , the signal of ¹³C-acetaldehyde and the signal of ¹³C-ethanol are detected by ¹³C-MR detection.

Another embodiment of the present invention is a composition as described above for use in a method for determining ADH activity, wherein the signal of ¹³C-acetaldehyde and optionally the signal of ¹³C-ethanol is/are detected by ¹³C-MR detection.

Still another embodiment of the present invention is a method for determining ALDH activity by ¹³C-MR detection using the composition of claim 1, wherein the signal of ¹³C-acetate and optionally the signal of ¹³C-ethanol and/or ¹³C-acetaldehyde is/are detected.

Another embodiment of the present invention is a method for determining ADH activity by ¹³C-MR detection using the composition of claim 1, wherein the signal of ¹³C-acetaldehyde and optionally the signal of ¹³C-ethanol is/are detected.

In a preferred embodiment of the invention said determination of ADH activity is carried out in tissue different from liver tissue.

In a preferred embodiment of the method the signal or signals are used to generate a metabolic profile.

In another embodiment of the method of the invention, the method is carried out *in vivo* or *in vitro* and the signal or signals is/are used in assessing the efficacy of potential drugs to alter ALDH or ADH activity.

In still another embodiment of the method of the invention, the method is carried out *in vivo* or *in vitro* and the signal or signals is/are used to assess response to treatment and/or treatment efficacy in diseased patients undergoing treatment for their disease.

In still another embodiment of the method of the invention, the method is carried out *in vivo* or *in vitro* and the signal or signals is/are used for identifying patients at risk of developing a disease and/or candidates for preventive measures to avoid development of a disease.

It is also described a method for determining ALDH activity by ¹³C-MR directly in the liver.

The composition according to the invention may, as mentioned above, be used as a contrast medium for *in vivo* ¹³C-MR detection, i.e. in living human or non-human animal beings. Further the composition may be used as an contrast medium for *in vitro* ¹³C-MR detection, e.g. in cell cultures or body samples, *ex vivo* tissue obtained from a biopsy or isolated organs, all of those dervied from a human or non-human animal body.

The terms "hyperpolarised" and "polarised" are used interchangeably hereinafter and denote a nuclear polarisation level in excess of 0.1%, more preferred in excess of 1% and most preferred in excess of 10%.

Hyperpolarisation of NMR active ¹³C-nuclei may be achieved by different methods which are for instance described in WO-A-98/30918, WO-A-99/24080 and WO-A-99/35508 and hyperpolarisation methods known in the art are polarisation transfer from a noble gas, "brute force", spin refrigeration, the parahydrogen method and dynamic nuclear polarisation (DNP).

One way of obtaining hyperpolarised ¹³C-ethanol is by polarisation transfer from a hyperpolarised noble gas, which is described in WO-A- 98/30918. Noble gases having non-zero nuclear spin can be hyperpolarised by the use of circularly polarised light. A hyperpolarised noble gas, preferably He or Xe, or a mixture of such gases, may be used to effect polarisation of ¹³C-nuclei. The hyperpolarised gas may be in the gas phase or it may be dissolved in a liquid/solvent, or the hyperpolarised gas itself may serve as a solvent. Alternatively, the gas may be condensed onto a cooled solid surface and used in this form, or allowed to sublime.

Another way for obtaining hyperpolarised ¹³C-ethanol is that polarisation is imparted to ¹³C-nuclei by thermodynamic equilibration at a very low temperature and high field. Hyperpolarisation compared to the operating field and temperature of the NMR spectrometer is effected by use of a very high field and very low temperature ("brute force"). The magnetic field strength used should be as high as possible, suitably higher than 1 T, preferably higher than 5 T, more preferably higher than 15 T or more and especially preferably 20 T or more. The temperature should be very low, e.g. 4.2 K or less, preferably 1.5 K or less, more preferably 1.0 K or less, especially preferably 100mK or less.

Another way for obtaining hyperpolarised ¹³C-ethanol is the spin refrigeration method. This method covers spin polarisation of a solid compound or system by spin refrigeration polarisation. The system is doped with, or intimately mixed with, suitable crystalline paramagnetic material such as Ni²⁺, lanthanide or actinide ions with a symmetry axis of order three or more. The instrumentation is simpler than required for DNP with no need for a uniform magnetic field since no resonance excitation field is applied. The process is carried out by physically rotating the sample around an axis perpendicular to the direction of the magnetic field. The prerequisite for this method is that the paramagnetic species has a highly anisotropic g-factor. As a result of the sample rotation, the electron paramagnetic resonance will be brought into contact with the nuclear spins, leading to a decrease in the nuclear spin temperature. Sample rotation is carried out until the nuclear spin polarisation has reached a new equilibrium.

To polarise a chemical entity, i.e. compound, by the DNP method, a composition comprising the compound to be polarised and a DNP agent is prepared which is then frozen and inserted into the DNP polariser for polarisation. After the polarisation, the frozen solid hyperpolarised composition is rapidly transferred into the liquid state, either by melting it or by dissolving it in a suitable dissolution medium. Dissolution is preferred and the dissolution process of a frozen hyperpolarised composition and suitable devices therefore are described in detail in WO-A-02/36005.

In order to obtain a high polarisation level in the compound to be polarised said compound and the DNP agent need to be in intimate contact during the DNP process. This is not the case if the composition crystallizes upon being frozen or cooled. To avoid crystallization, either glass formers need to be present in the composition or compounds need to be chosen for polarisations that do not crystallize upon being frozen, but rather form a glass.

If the hyperpolarised ¹³C-²H ethanol used in the method of the invention is obtained by DNP, the sample to be polarised, i.e. ¹³C ethanol and a DNP agent may further comprise a paramagnetic metal ion. The presence of paramagnetic metal ions in the composition to be polarised by DNP has been found to result in increased polarisation levels, as described in detail in WO-A2-2007/064226. Hence in a preferred embodiment the composition to be polarised comprises [1-¹³C, U-²H₅]ethanol, a trityl radical and optionally a paramagnetic metal ion. When said paramagnetic metal ion is present this is preferably present in the form of gadoteric acid which comprises Gd³⁺. Such MR imaging medium is preferably obtained by dynamic nuclear polarisation.

As mentioned earlier, compositions according to the invention may be used as contrast medium for *in vivo* ALDH or ADH activity determination by ¹³C-MR determination, i.e. in living human or non-human beings. For this purpose, the contrast medium is provided as a composition that is suitable for being administered to a living human or non-human animal body. Such a contrast medium preferably comprises, in addition to the MR active agent, [1-¹³C, U-²H₅]ethanol, an aqueous carrier, preferably a physiologically tolerable and pharmaceutically acceptable aqueous carrier like water, a buffer solution or saline. Such an composition may further comprise conventional pharmaceutical or veterinary carrier or exipients, e.g. formulation aids such as are conventional for diagnostic compositions in human or veterinary medicine.

In one embodiment, the method of the invention is carried out *in vitro* and the information obtained is used in assessing the efficacy of potential drugs that alter ALDH activity, e.g. in a drug discovery and/or screening process. In such an embodiment, the method of the invention may be carried out in suitable cell cultures or tissue. The cells or the tissue is contacted with the potential drug and ALDH or ADH activity is determined by ¹³C-MR detection according to the method of the invention. Information about the efficacy of the potential drug may be obtained by comparing the ALDH or ADH activity of the treated cells or tissue with the ALDH or ADH activity of non-treated cells or tissue. Alternatively, the variation of ALDH or ADH activity may be determined by determining the ALDH or ADH activity of cells or tissue before and after treatment. Such a drug efficacy assessment may be carried out on for instance microplates, which would allow parallel testing of various potential drugs and/or various doses of potential drugs and thus would make this suitable for high-throughput screening.

In another preferred embodiment, the method of the invention is carried out *in vivo* and the information obtained is used in assessing the efficacy of potential drugs that alter ALDH or ADH activity *in vivo.* In such an embodiment, the method of the invention may be carried out in, for instance, test animals or volunteers in a clinical trial. To the test animal or the volunteer a potential drug is administered and ALDH or ADH activity is determined by ¹³C-MR detection according to the method of the invention. Information about the efficacy of the potential drug may be obtained by determining the variation of ALDH or ADH activity before and after treatment, e.g. over a certain period with repeated treatment. Such a drug efficacy assessment may be carried out in pre-clinical research (test animals) or in clinical trials.

In another embodiment, the method of the invention is carried out *in vivo* or *in vitro* and the information obtained is used to assess response to treatment and/or to determine treatment efficacy in patients undergoing treatment for their disease.

As stated earlier, the information obtained by the method of the invention may be used in a subsequent step for various purposes. Another purpose may be to gain insight into disease states, i.e. identify patients at risk, early detection of diseases, evaluating disease progression, severity and complications related to a disease.

In yet another embodiment, the method of the invention is carried out *in vivo* or *in vitro* and the information obtained is used to monitor progression of a disease. This may be useful for diseases or disorders where the disease has not progessed to a level where treatment is indicated or recommended, e.g. because of severe side-effects associated with said treatment. In such a situation the choice of action is "watchful waiting", i.e. the patient is closely monitored for disease progression and early detection or deterioration. In this embodiment, the method of the invention may be used to determine the initial ALDH or ADH activity and to make subsequent ALDH or ADH activity determinations over a period of time at a certain frequency. It can be expected that a decrease or change in ALDH or ADH activity may indicate progress or worsening of a disease and the said decrease can be used by the physician to decide on commencement of treatment. To carry out the method of the invention for the above-mentioned purpose *in vitro* does of course require that suitable samples from a patient under treatment are obtainable, e.g. tissue samples or body samples.

Further, the imaging medium according to the invention may be used as contrast medium for *in vitro* ALDH or ADH activity determination by ¹³C-MR detection, i.e. in cell cultures or body samples, *ex vivo* tissues such as biopsy tissue or isolated organs. For this purpose, the composition is provided as a composition that is suitable for being added to, for instance, cell cultures, body samples, ex vivo tissues like biopsy tissue or isolated organs. Such a composition preferably comprises in addition to the MR active agent, deuterated ¹³C ethanol, a solvent which is compatible with and used for *in vitro* cell or tissue assays, for instance DMSO or methanol or solvent mixtures comprising an aqueous carrier and a non aqueous solvent, for instance mixtures or DMSO and water or a buffer solution or methanol and water and a buffer solution. As is apparent for a skilled person, pharmaceutically acceptable carriers, excipients and formulation aids may be present in such a composition but are not required for such a purpose.

If the composition used in the method of the invention is used for *in vivo* dtermination of ALDH2 activity, i.e. in a living human or non-human animal body, said medium is preferably administered to said body by any route known in the art, preferable parenterally, more preferable intravenously. Generally, the body under examination is positioned in an MR magnet. Dedicated ¹³C-MR and ¹H-MR or double tuned ¹³C/¹H RF-coils are positioned to cover the area of interest. Exact dosage and concentration of the composition will depend upon a variety of factors such as toxicity and the administration route. At specified time after administration, an MR imaging sequence is applied preferably one that encodes the volume of interest in a combined frequency and spatially selective way. The exact time of applying an MR sequence is highly dependent on the volume of interest and the species.

The ALDH or ADH activity map or measurement generated in the preferred embodiment of the use according to the invention is indicative for the ALDH or ADH activity of the body, part of the body, cells, tissue body sample etc. under examination and said information obtained may be used in a subsequent step for various purposes.

### Brief description of the drawings:

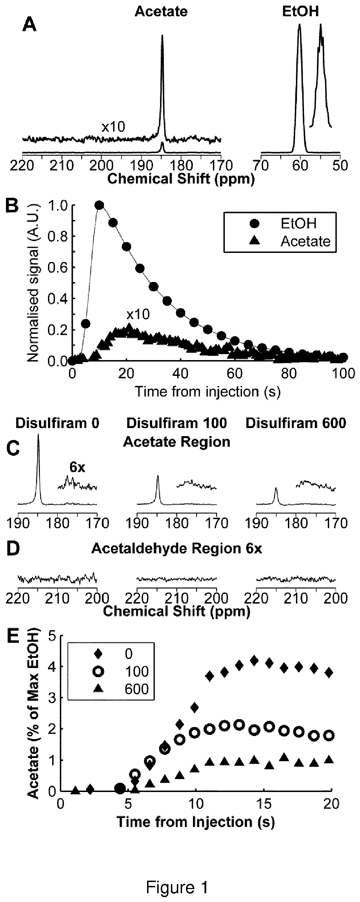

**Figure 1** shows:
   (a) Typical ethanol (EtOH) and acetate ¹³C signals acquired 20 s after i.v. injection of hyperpolarised [1-¹³C, U-²H₅]ethanol into a mouse. The surface receive coil was placed over the liver. The inserts show a 10x vertical scale expansion of the acetate signal and a resolution-enhanced ethanol signal showing the splitting due to carbon-deuterium coupling.
   (b) Time dependence of the ethanol and acetate signals following ethanol injection. The amplitude of the acetate signal has been multiplied 10x.
   (c-e) The effect of disulfiram treatment on acetate signal intensity. The [1-¹³C]acetate (175 - 190 ppm) (c) and [1-¹³C] acetaldehyde (200 - 215 ppm) (d) regions of the spectra are shown from animals treated with the indicated disulfiram concentrations (mg/kg body weight) -24 h prior to the MR experiment.
   (e) Time dependence of the acetate signal intensity following disulfiram treatment at the indicated concentrations (mg/kg body weight).

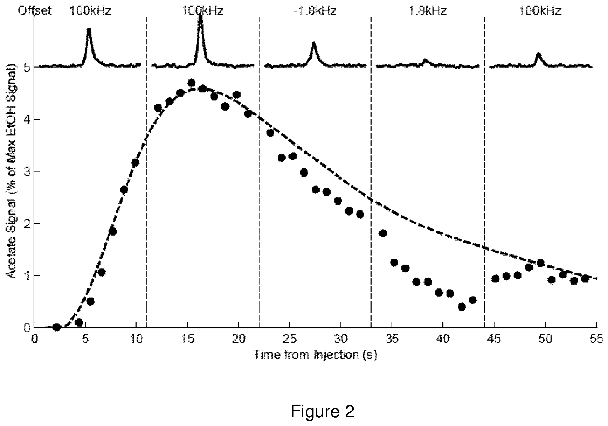

**Figure 2** shows:
   Time dependence of the acetate signal, where a saturation pulse (at the specified frequency offset from the acetate signal) was applied prior to each acquisition. The last spectrum from each section of nine acquisitions is also shown. The dotted line shows the expected signal development based on the first two sections. The acetaldehyde resonance is at +1.8 kHz from the acetate resonance.

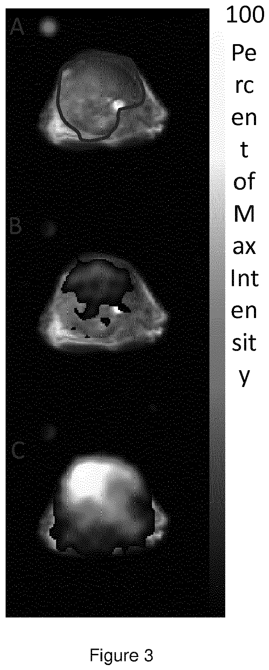

**Figure 3** shows:
   Chemical shift images of urea (a), acetate (b) and ethanol (c) superimposed on a reference ¹H image (the location of the liver is circled in a). The ethanol CSI was collected 30 s later than CSI of the acetate and urea signals. All images were normalized to the maximum ethanol signal intensity.

### Examples

### Method used

**¹³C T₁ measurements *in vitro*:** Measurements were made at 11 T (Bruker Avance II⁺ 500 MHz spectrometer) using an inversion recovery sequence in samples containing approximately 10 mM [1-¹³C] ethanol or 1 M ethanol, 10% D₂O and 4% BSA (Sigma Aldrich), buffered with PBS to pH 7.5. A TR of 300 seconds and 16 delays between 1 and 200 seconds were used for measurements with [1-¹³C, U-²H₅] ethanol and a TR of 50 seconds and 16 delays between 0.5 and 50 seconds were used for measurements of the natural abundance ¹³C signal from 1M ethanol.

### Example 1: Hyperpolarisation of [1-¹³C] ethanol:

A 2:3 glycerol: [1-¹³C] ethanol mixture, containing 15 mM trityl radical (OX063: tris (8-carboxy-2,2,6,6-tetra-(hydroxyethyl)-benzo-[1,2-4,50]-bis-(1,3)-dithiole-4-yl)-methyl, sodium salt; GE Healthcare, Little Chalfont, UK) and 1.5 mM gadoteric acid (DOTAREM; Guebert, Roissy, France), was polarized for approximately 120 minutes in a 3.35 T Hypersense polarizer (Oxford Instruments, Abingdon, Oxfordshire, UK) using irradiation at 94.124 GHz. The dissolution buffer (PBS, pH 7.5, 100 mg/L EDTA) was heated to 180°C and pressurized to 10 bar before dissolving the frozen polarized samples. The dissolved sample contained 110 mM [1-¹³C] ethanol. For measurements of the liquid state polarization a sample of [1-¹³C] ethanol was polarized and dissolved as described above. Two mL of the sample, containing approximately 60 mM [1-¹³C] ethanol were injected into 2 mL of PBS buffer, pH 7.4 in a 10 mm NMR tube placed inside a vertical bore 9.4 T NMR spectrometer with a broadband probe tuned to ¹³C (Varian NMR Instruments, Palo Alto, CA, U.S.). Immediately following injection and approximately 9 seconds after dissolution, 180 single transient spectra, with a nominal pulse flip angle of 6° and 8 kHz spectral width, were acquired with a TR of 1 second. Three 90° pulses were then applied to eliminate the remaining hyperpolarisation. A ¹³C spectrum (the sum of 128 transients) was then acquired using a 6° pulse, a spectral width of 8 kHz, and a TR of 300 seconds. The temperature of the sample was kept at 37°C. The liquid state polarization was calculated from the ratio of the maximal integrated intensity of the hyperpolarised [1-¹³C] ethanol signal to the integrated intensity of the thermal equilibrium signal corrected for the number of transients.

### Example 2 - Animal experiments:

All animal experiments were approved by local ethical review committees and performed in accordance with the Animals (Scientific Procedures) Act of 1986. Female CD1 mice (6-8 weeks old) were used in all experiments. Animals were anaesthetized using 3 % isoflurane vapor (Isoflo, Abbotts Laboratories Ltd, Maidenhead, UK) in an oxygen/air mixture. Anesthesia was maintained during the MR experiments with 1-2 % isoflurane vapor in an oxygen/air mixture delivered via a face mask.

***In vivo* NMR spectroscopy:** A catheter was inserted in a tail vein and the mouse placed in a heated cradle. Throughout the experiment animal breathing rate and core body temperature were monitored using a Biotrig physiological monitor (Small Animal Instruments, Stony Brook, NY), which were within the range of 60-100 bpm and 36-37°C, respectively. A 20 mm diameter surface coil (Rapid Biomedical GmbH, Rimpar, Germany) was placed over the liver and the entire assembly placed in a ¹³C/¹H volume coil (Rapid Biomedicals, Germany), in a 7T horizontal bore magnet (Varian, Palo Alto, CA). Livers were localized in transverse ¹H images, acquired using a spin-echo pulse sequence (TR, 1.5 s; echo time (TE), 10 ms; field of view, 40 mm x 40 mm; data matrix, 128 x 128; slice thickness, 2 mm; 15 slices). Immediately after dissolution, ¹³C spectroscopic acquisitions were started and 10 µl of the dissolved sample per g body weight, typically 240 µL in total, were injected via the tail vein over a period of 2 seconds. Single transient spectra (sweep width 6 kHz, TE 280 ms) from the entire sensitive volume of the surface coil were acquired using a frequency-selective excitation pulse (450 µs 3-lobe sinc pulse) centered either at 180 ppm (the acetate region of the spectrum) or at 60 ppm (the ethanol region of the spectrum) with a nominal flip angle of 10°. A series of ethanol and acetate spectra were acquired from one animal, where four acetate spectra were acquired followed by a single ethanol spectrum (TR 1.0s) and this pattern was repeated over a period of 120 s (Figure 1). In animals in which ALDH2 was inhibited with disulfiram, spectra were collected using a repeated acquisition pattern, where nine acetate spectra were acquired followed by a single ethanol spectrum (TR 1.1 s). Within each repeat, every spectral acquisition was preceded by a saturation pulse (1s hard pulse with a B₁ field of 100 Hz) at 100 kHz (control saturations 1 and 2 (see Figure 2)), -1840Hz (control saturation 3), 1840 Hz (saturation at the acetaldehyde resonance frequency) or at 100 kHz (control saturation 4) from the acetate resonance frequency. In three animals, two chemical shift images (FOV 40x40mm, 32x32 data matrix, TR 30 ms, TE 0.58 ms) were acquired, starting 15 s after ethanol injection, where the images were acquired first from acetate and then ethanol. All data were phase and baseline corrected and the signal integrals for ethanol and acetate measured.

### Example 3 - Disulfiram dose-response experiments:

Animals were given 100 mg/kg or 600 mg/kg body weight of disulfiram as a suspension in 5% w/v of *gum arabicum,* by oral gavage (Spielman, D.M., Mayer, D., Yen, Y.F., Tropp, J., Hurd, R.E., Pfefferbaum, A., "In vivo measurement of ethanol metabolism in the rat liver using magnetic resonance spectroscopy of hyperpolarised [1-13C]pyruvate.", Magn.Reson.Med. 2009; 62: 307-313). ¹³C MRS experiments were performed approximately 24 hours after drug administration. In some experiments, shortly after completion of MRS measurements, animals were killed and their livers rapidly excised, freeze-clamped and stored at - 80°C before homogenization and enzyme assay.

**Measurement of blood ethanol level:** An appropriate volume of [2-¹³C] ethanol, polarized and dissolved to the same concentration as [1-¹³C] ethanol, was injected into the tail vein of animals that had been anesthetized for 30-45 minutes. Blood was withdrawn by cardiac puncture, which began 75 seconds after the start of ethanol injection and was completed within 30 seconds. Individual blood samples were transferred into separate BD Microtainer FE micro-collection tubes (BD, Franklin Lakes, NJ, US), centrifuged at 800 g and the plasma removed and frozen in liquid nitrogen. For NMR spectroscopy, plasma samples were thawed and diluted in 200 mM PBS in D₂O, pH 7.5. The buffer contained 100 mM TMSP (2,2,3,3-D4 sodium-3-trimethylsilylpropionate) as an intensity and chemical shift standard (Cambridge Isotope Laboratories, Inc. Andover MA, US). ¹³C spectra (the sum of 1024 transients) were acquired under fully relaxed conditions at 125 MHz (Bruker Avance II⁺ 500 MHz spectrometer) into 25,294 data points using a 90° pulse, a sweep width of 5 kHz, and a TR of 35 seconds. Integrated intensities of the 2-¹³C ethanol and 2-¹³C acetate resonances were normalized to that of TMSP and multiplied by the methyl ¹³C concentration in 100 mM TMSP (3.33 mM) in order to convert to acetate or ethanol concentrations.

**Liver Tissue Extraction:** Freeze-clamped livers were homogenized using a manual Potter S homogenizer (Sartorius, Epsom, UK) in 7 volumes (mL per gram wet weight of liver) of ice-cold extraction buffer (50 mM Tris-HCl, pH 7.4 containing 0.25 M sucrose, 0.1 % v/v 2-mercaptoethanol, 1 mM EDTA and protease inhibitor cocktail (Roche)). Ten strokes of 5 seconds (each followed by a 5 second pause) at 1000 r.p.m. were used. Homogenates were centrifuged at 800 *g* (Eppendorf 5415D benchtop centrifuge) at 4°C. The supernatant was extracted by the addition of 20 % v/v of extraction buffer containing 5% v/v of Triton X-100. The extracts were centrifuged at 16200 g at 4°C to remove debris. Supernatants were kept on ice and used for enzyme assays.

**ALDH2 activity assay:** The assay mixture containing 0.5 mM NAD⁺, 0.1 % v/v of 2-mercaptoethanol, 200 mM KCl in 100 mM sodium pyrophosphate buffer, pH 9.0. The sample of liver extract was pre-incubated at room temperature for 20 minutes and the reaction initiated by the addition of propionaldehyde to a final concentration of 20 µM. At this low aldehyde concentration the assay measures predominantly ALDH2 activity (Klyosov, A.A., Rashkovetsky, L.G., Tahir, M.K., Keung, W.M., "Possible role of liver cytosolic and mitochondrial aldehyde dehydrogenase in acetaldehyde metabolism.", Biochemistry 1996; 35: 4445-4456; Eriksson, C.J., Marselos, M., Koivula, T., "Role of cytosolic liver aldehyde dehydrogenase in the oxidation of acetaldehyde during ethanol metabolism in vivo.", Biochem.J. 1975; 152: 709-712). The formation of NADH was measured for 5 minutes after substrate addition as an increase in absorbance at 340 nm using a Tecan Sunrise 96 well plate reader. One unit of ALDH activity is defined as the amount of enzyme that oxidizes 1 µmol of propionaldehyde (and reduces 1 µmol of NAD⁺) per minute. All assays were carried out at 25°C.

The ¹³C T₁ of perdeuterated ethanol ([1-¹³C, U-²H₅]ethanol) in a PBS buffer, pH 7.5, containing 4% BSA was 56 s s (n= 2; 56.6 s and 55.3 s) and 9.9 s(n= 2; 9.8 s and 10.0 s) for natural abundance ¹³C in the protonated form. The T₁ calculated from the decay of the polarization in [1-¹³C, U-²H₅]ethanol was 53.7 +/- 3.2 s (SD, n=3) The polarization buildup time constant of dynamic nuclear polarization of the deuterated form was 41.2 ± 5.5 minutes (n = 3) and the liquid state polarization was 14.1 ± 3.3 % when measured approximately 9 seconds after dissolution.

Hyperpolarised [1-¹³C, U-²H₅] ethanol (110 mM) was injected into mice via a tail vein catheter (10 µl/g body weight) and a series of ¹³C spectra were acquired using a surface coil placed over the liver (Figure 1). These showed oxidation of [1-¹³C, U-²H₅] ethanol to produce [1-¹³C] acetate (∼185 ppm) (Figure 1 a-c). The [1-¹³C] acetaldehyde intermediate, at 207.4 ppm, was not observed, even following treatment of the animals with the irreversible ALDH2 inhibitor, disulfiram (Figure 1 d). The metabolites downstream from [1-¹³C] acetate; [1-¹³C] acetylcarnitine (at 175 ppm) (Jensen, P.R., Peitersen, T., Karlsson, M., t'Zandt, R., Gisselsson, A., Gisselsson, A., Hansson, G., Meier, S., Lerche, M.H., "Tissue-specific short chain fatty acid metabolism and slow metabolic recovery after ischemia from hyperpolarised NMR in vivo.", J.Biol.Chem. 2009; 284: 36077-36082; Bastiaansen, J.A.M., Cheng, T., Mishkovsky, M., Duarte, J.M.N., Comment, A., Gruetter, R., "In vivo enzymatic activity of acetylCoA synthetase in skeletal muscle revealed by 13C turnover from hyperpolarised [1-13C]acetate to [1-13C]acetylcarnitine." Biochim.Biophys.Acta 2013; 1830: 4171-4178), and H¹³CO⁻₃ (at 164 ppm) were, in general not detected, although in some animals resonances were observed between 175 and 180 ppm (Figure 1 c).

Treatment of the animals with disulfiram decreased the maximum acetate signal (normalized to the maximum ethanol signal) by approximately 43 % at a dose of 100 mg/kg (n=1) and by 79 ± 8 % (SD, n=3) at 600 mg/kg body weight (Figure 1c & e). The ALDH2 activity in extracts of livers from control animals was 0.37 ± 0.07 units per gram wet weight (U/gww) (SD, n = 3) and 0.12 ± 0.10 U/gww (SD, n = 3) in animals 24 hours after treatment with 600 mg / kg body weight disulfiram, a decrease of 68%. Introduction of a saturation pulse at the resonance frequency of the unobserved [1-¹³C] acetaldehyde resulted in a rapid decrease in the acetate signal intensity and subsequent recovery when saturation was stopped, demonstrating that the acetate was produced via acetaldehyde (Figure 2). Chemical shift images showed that the ethanol and acetate signals were mainly from the liver (Figure 3).
¹³C MRS measurements on plasma samples withdrawn 75 seconds after the start of [2-¹³C] ethanol injection gave an ethanol concentration of 1.73 ± 0.48 mM (SD, n = 3) and an [1-¹³C] acetate concentration of 0.93 ± 0.29 mM (SD, n = 3).

### Discussion of results

The disulfiram - sensitive, mitochondrial ('low Kₘ') isoform of ALDH (ALDH2) is primarily responsible for acetaldehyde oxidation in the hepatocyte during ethanol metabolism and accounts for approximately 60 - 80% of acetaldehyde oxidation in the liver in both healthy humans (Forte-McRobbie, C.M., Pietruszko, R., "Aldehyde dehydrogenase content and composition of human liver.", Alcohol 1985; 2: 375-381) and in rodents (Klyosov, A.A., Rashkovetsky, L.G., Tahir, M.K., Keung, W.M., "Possible role of liver cytosolic and mitochondrial aldehyde dehydrogenase in acetaldehyde metabolism.", Biochemistry 1996; 35: 4445-4456). The other enzymes, including the cytosolic ('high Km') ALDH1 isoform, account for the remaining 20 - 40 % (Klyosov, A.A., Rashkovetsky, L.G., Tahir, M.K., Keung, W.M., "Possible role of liver cytosolic and mitochondrial aldehyde dehydrogenase in acetaldehyde metabolism.", Biochemistry 1996; 35: 4445-4456; Eriksson, C.J., Marselos, M., Koivula, T., "Role of cytosolic rat liver aldehyde dehydrogenase in the oxidation of acetaldehyde during ethanol metabolism in vivo.", Biochem.J. 1975; 152: 709-712; Forte-McRobbie, C.M., Pietruszko, R., "Aldehyde dehydrogenase content and composition of human liver.", Alcohol 1985; 2: 375-381 and Svanas, G.W., Weiner, H., "Aldehyde dehydrogenase activity as the rate-limiting factor for acetaldehyde metabolism in rat liver." Arch.Biochem.Biophys. 1985; 236: 36-46). Decreasing ALDH activity in this study by 68%, using the irreversible ALDH2 inhibitor disulfiram (Klyosov, A.A., Rashkovetsky, L.G., Tahir, M.K., Keung, W.M., "Possible role of liver cytosolic and mitochondrial aldehyde dehydrogenase in acetaldehyde metabolism.", Biochemistry 1996; 35: 4445-4456 and Tottmar, O., Marchner, H.,"Disulfiram as a tool in the studies on the metabolism of acetaldehyde in rats.", Acta Pharmacol. Toxicol. (Copenh) 1976; 38: 366-375), reduced the acetate signal intensity by ∼79%, and implies, therefore, that the rate of acetate production from hyperpolarised [1-¹³C, U-²H₅] ethanol in the liver is determined primarily by ALDH2 activity. This reduction in ALDH2 activity assayed in tissue extracts agrees well with a decrease of approximately 67 % measured in rat liver 24 h after administration of 600 mg/kg disulfiram (Tottmar, O., Marchner, H.,"Disulfiram as a tool in the studies on the metabolism of acetaldehyde in rats.", Acta Pharmacol. Toxicol. (Copenh) 1976; 38: 366-375). Although the production of acetaldehyde from ethanol was not observed, the level of acetaldehyde being too low to detect, applying a saturation pulse at the acetaldehyde resonance frequency and observing a decrease in the acetate signal intensity confirmed that the production of acetate from [1-¹³C, U-²H₅] ethanol proceeded via acetaldehyde. This magnetization transfer experiment may provide an alternative method for assessing ALDH activity.

The observation that flux of hyperpolarised ¹³C label from [1-¹³C, U-²H₅] ethanol is strongly dependent on ALDH2 activity, and thus can be used to assess changes in the activity of this enzyme in liver, further implies that the delivery of ethanol and its transport into the cell and the activity of ADH have little influence on the rate of acetate production. This agrees with *ex vivo* work demonstrating that the activity of mitochondrial ALDH governs the rate of acetaldehyde oxidation during ethanol metabolism in rat liver slices (Svanas, G.W., Weiner, H., "Aldehyde dehydrogenase activity as the rate-limiting factor for acetaldehyde metabolism in rat liver.", Arch.Biochem.Biophys. 1085; 236: 36-46). The amphipathic nature of ethanol means that it can freely diffuse from the bloodstream into tissues and therefore its utilization is less likely to be limited by transport into cells when compared to other hyperpolarised ¹³C-labelled tracers that require facilitated membrane transport (Witney, T.H., Kettunen, M.I., Brindle, K.M., "Kinetic modeling of hyperpolarised 13C label exchange between pyruvate and lactate in tumor cells.", J.Biol.Chem. 2011; 286: 24572-24580). Assuming that water accounts for approximately 74% of body weight in rodents, injection of 10 µl per g body weight of 110 mM ethanol would be expected to give approximately 1.5 mM of ethanol at equilibrium, with approximately equal concentrations in intra- and extracellular water (Xiang, Y., Shen, J., "In vivo detection of intermediate metabolic products of [1-13C]ethanol in the brain using 13C MRS."NMR Biomed. 2011; 24: 1054-1062; Trippodo, N.C., Walsh, G.M., Frolich, E.D., "Fluid volumes during onset of spontaneous hypertension in rats.", Am.J.Physiol. 1978; 235: H52-55). Consistent with this expectation a blood concentration of 1.73 mM at 75 s after injection of 10 µl per g body weight of 110 mM ethanol was measured.

Per-deuteration of the molecule extended the T₁ relaxation time by a factor of ∼5, to 55 s, making possible imaging of the hyperpolarised molecule and its subsequent metabolism (Figure 3). Although deuteration will affect the ADH-catalyzed oxidation of ethanol to acetaldehyde, through a primary kinetic isotope effect (Brooks, R.I., Shore, J.D., "Effect of substrate structure on the rate of the catalytic step in the liver alcohol dehydrogenase mechanism.", Biochemistry 1971; 10: 3855-3858), it will not affect the subsequent oxidation to acetate, catalyzed by ALDH, where there is no such effect (Ni, L., Sheikh, S., Weiner, H., "Involvement of glutamate 399 and lysine 192 in the mechanism of human liver mitochondrial dehydrogenase.", J.Biol.Chem. 1997; 272: 18823-18826).

In summary, the oxidation of ethanol to acetate *in vivo* in real time using hyperpolarised [1-¹³C, U-²H₅] ethanol has been measured and it has been demonstrated that in liver this flux is determined predominantly by ALDH2 activity. Since intravenous ethanol administration is a clinically approved medical procedure in, for example, the treatment of ethylene glycol poisoning (Scalley, R.D., Ferguson, D.R., Piccaro, J.C., Smart, M.L., Archie, T.E., "Treament of ethylene glycol poisoning." Am.Fam.Physician 2002; 66: 807-812) hyperpolarised [1-¹³C, U-²H₅]ethanol could be translated to the clinic as means of assessing ALDH2 -targeted therapy. A limitation of the method is the requirement that the tissue contain ADH activity, so for example it could not be used directly in heart muscle to measure ALDH2 activity as it lacks ADH. Nevertheless measurements in liver could still be used to assess the efficacy of drugs targeted at ALDH2. Measurements with hyperpolarised [1-¹³C] acetaldehyde would remove this requirement for the presence of ADH activity, however these are likely to be limited by unacceptable toxicity of the aldehyde (Von Burg, R., Stout, T., "Acetaldehyde.", J.Appl.Toxicol. 1991; 11: 373-376). In addition, hyperpolarised [1-¹³C, U-²H₅] ethanol also offers a new avenue for elucidating certain aspects of ethanol metabolism, for example the possibility of oxidation of ethanol by the brain, which until now has been limited to *ex vivo* studies (Wang, J., Du, H., Jiang, L., Ma, X., de Graaf, R.A., Behar, K.L., Mason, G.F., "Oxidation of ethanol in the rat brain and effects associated with chronic ethanol exposure." , Proc.Natl.Acad.Sci USA 2013; 110:14444-14449).

## Claims

1. A composition comprising hyperpolarised [¹³C]-ethanol which ethanol is deuterated on at least one hydrogen atom, wherein the hyperpolarisation is achieved on the NMR active ¹³C-nuclei.

2. The composition of claim 1, wherein said composition is a MR contrast medium.

3. The composition of claim 1, wherein carbon 1, carbon 2 or both carbon 1 and carbon 2 is/are ¹³C-labelled.

4. The composition of claim 1, wherein said ethanol is deuterated on at least two hydrogen atoms, at least three hydrogen atoms, at least four hydrogen atoms or at five hydrogen atoms.

5. The composition of claim 1, wherein the deuterated [¹³C]-ethanol is [1-¹³C, U-²H₅].

6. The composition of claim 1 for use in a method for determining ALDH activity; wherein the signal of [¹³C]-acetate and optionally the signal of ¹³C-ethanol and/or ¹³C-acetaldehyde is/are detected by ¹³C-MR detection.

7. The composition of claim 1 for use in a method for determining ALDH2 activity; wherein the signal of [¹³C]-acetate and optionally the signal of ¹³C-ethanol is/are detected by ¹³C-MR detection.

8. The composition of claim 1 for use in a method for determining ADH activity; wherein the signal of ¹³C-acetaldehyde and optionally the signal of ¹³C-ethanol is/are detected by ¹³C-MR detection.

9. The composition of claim 6 or 8, for use in an *in vivo* method.

10. The composition of claim 6 or 8, for use in an *in vitro* method.

11. A method for determining ALDH activity by ¹³C-MR detection using the composition of claim 1 wherein the signal of [¹³C]-acetate and optionally the signal of ¹³C-ethanol and/or ¹³C-acetaldehyde is/are detected.

12. A method of claim 11, for determining ALDH2 activity by ¹³C-MR detection using the composition of claim 1 wherein the signal of [¹³C]-acetate and optionally the signal of ¹³C-ethanol is/are detected.

13. A method for determining ADH activity by ¹³C-MR detection using the composition of claim 1, wherein the signal of ¹³C-acetaldehyde and optionally the signal of ¹³C-ethanol is/are detected.

14. The method of claim 11 or 13 or the composition of claim 6 or 8, which is carried out *in vivo* or *in vitro* and wherein the signal or signals is/are used in assessing the efficacy of potential drugs that alter ALDH activity.

## Patentansprüche

1. Zusammensetzung umfassend hyperpolarisiertes [¹³C]-Ethanol, welches Ethanol an mindestens einem Wasserstoffatom deuteriert ist, wobei die Hyperpolarisation an dem NMR-aktiven ¹³C-Kern erzielt ist.

2. Zusammensetzung nach Anspruch 1, wobei die Zusammensetzung ein MR-Kontrastmedium ist.

3. Zusammensetzung nach Anspruch 1, wobei Kohlenstoff 1, Kohlenstoff 2 oder sowohl Kohlenstoff 1 als auch Kohlenstoff 2 ¹³C-markiert sind.

4. Zusammensetzung nach Anspruch 1, wobei das Ethanol an mindestens zwei Wasserstoffatomen, mindestens drei Wasserstoffatomen, mindestens vier Wasserstoffatomen oder an fünf Wasserstoffatomen deuteriert ist.

5. Zusammensetzung nach Anspruch 1, wobei das deuterierte [¹³C]-Ethanol [1-¹³C, U-²H₅] ist.

6. Zusammensetzung nach Anspruch 1 zur Verwendung in einem Verfahren zur Bestimmung der ALDH-Aktivität; wobei das Signal des [¹³C]-Acetats und gegebenenfalls das Signal des ¹³C-Ethanols und/oder des ¹³C-Acetaldehyds durch ¹³C-MR-Nachweis nachgewiesen werden.

7. Zusammensetzung nach Anspruch 1 zur Verwendung in einem Verfahren zur Bestimmung der ALDH2-Aktivität; wobei das Signal des [¹³C]-Acetats und gegebenenfalls das Signal des ¹³C-Ethanols durch ¹³C-MR-Nachweis nachgewiesen werden.

8. Zusammensetzung nach Anspruch 1 zur Verwendung in einem Verfahren zur Bestimmung der ADH-Aktivität; wobei das Signal des ¹³C-Acetaldehyds und gegebenenfalls das Signal des ¹³C-Ethanols durch ¹³C-MR-Nachweis nachgewiesen werden.

9. Zusammensetzung nach Anspruch 6 oder 8, zur Verwendung in einem *in vivo*-Verfahren.

10. Zusammensetzung nach Anspruch 6 oder 8, zur Verwendung in einem *in vitro*-Verfahren.

11. Verfahren zur Bestimmung der ALDH-Aktivität durch ¹³C-MR-Nachweis unter Verwendung der Zusammensetzung nach Anspruch 1, wobei das Signal des ¹³C]-Acetats und gegebenenfalls das Signal des ¹³C-Ethanols und/oder des ¹³C-Acetaldehyds nachgewiesen werden.

12. Verfahren nach Anspruch 11, zur Bestimmung der ALDH2-Aktivität durch ¹³C-MR-Nachweis unter Verwendung der Zusammensetzung nach Anspruch 1, wobei das Signal des [¹³C]-Acetats und gegebenenfalls das Signal des ¹³C-Ethanols nachgewiesen werden.

13. Verfahren zur Bestimmung der ADH-Aktivität durch ¹³C-MR-Nachweis unter Verwendung der Zusammensetzung nach Anspruch 1, wobei das Signal des ¹³C-Acetaldehyds und gegebenenfalls das Signal des ¹³C-Ethanols nachgewiesen werden.

14. Verfahren nach Anspruch 11 oder 13 oder Zusammensetzung nach Anspruch 6 oder 8, welches *in vivo* oder *in vitro* durchgeführt wird, und wobei das Signal oder die Signale bei der Beurteilung der Wirksamkeit von die ALDH-Aktivität verändernden potentiellen Arzneimitteln verwendet werden.

## Revendications

1. Composition comprenant [¹³C]-éthanol hyperpolarisé, dans laquelle l'éthanol est deutérié sur au moins un atome d'hydrogène, ladite hyperpolarisation étant obtenue sur le noyau de NMR actif ¹³C.

2. Composition selon la revendication 1, dans laquelle ladite composition est un milieu de contraste MR.

3. Composition selon la revendication 1, dans laquelle le carbone 1, le carbone 2 ou les deux carbone 1 et carbone 2 sont marqués par ¹³C.

4. Composition selon la revendication 1, dans laquelle ledit éthanol est deutérié par au moins deux atomes d'hydrogène, au moins trois atomes d'hydrogène, au moins quatre atomes d'hydrogène ou par cinq atomes d'hydrogène.

5. Composition selon la revendication 1, dans laquelle [¹³C]-éthanol deutérié est [1-¹³C, U-²H₅].

6. Composition selon la revendication 1 utilisable dans un procédé pour déterminer l'activité de l'ALDH ; où le signal de [¹³C]-acétate et éventuellement le signal de ¹³C-éthanol et / ou de ¹³C-acétaldéhyde est / sont détectés par la détection de ¹³C-MR.

7. Composition selon la revendication 1 utilisable dans un procédé pour déterminer l'activité de l'ALDH2 ; où le signal de [¹³C]-acétate et éventuellement le signal de ¹³C-éthanol est / sont détectés par la détection de ¹³C-MR.

8. Composition selon la revendication 1 utilisable dans un procédé pour déterminer l'activité de l'ADH ; où le signal de ¹³C-acétaldéhyde et éventuellement le signal de ¹³C-éthanol est / sont détectés par la détection de ¹³C-MR.

9. Composition selon la revendication 6 ou 8, à utiliser dans un procédé *in vivo.*

10. Composition selon la revendication 6 ou 8, à utiliser dans un procédé *in vitro.*

11. Procédé pour déterminer l'activité de l'ALDH par détection de ¹³C-MR en utilisant la composition selon la revendication 1; où le signal de [¹³C]-acétate et éventuellement le signal de ¹³C-éthanol et / ou de ¹³C-acétaldéhyde est / sont détectés.

12. Procédé selon la revendication 11, pour déterminer l'activité de l'ALDH2 par la détection de ¹³C-MR en utilisant la composition selon la revendication 1, où le signal de [¹³C]-acétate et éventuellement le signal de ¹³C-éthanol est / sont détectés.

13. Procédé pour déterminer l'activité de l'ADH par la détection de ¹³C-MR en utilisant la composition selon la revendication 1, où le signal de ¹³C-acétaldéhyde et éventuellement le signal de ¹³C-éthanol est / sont détectés.

14. Procédé selon la revendication 11 ou 13 ou la composition de la revendication 6 ou 8, qui est effectué *in vivo* ou *in vitro* où le ou les signaux sont utilisés pour évaluer l'efficacité de médicaments potentiels qui altèrent l'activité de l'ALDH.
